# EUROPEAN PATENT SPECIFICATION

(11) **EP 1 899 022 B1**
(45) Date of publication and mention of the grant of the patent: **09.10.2013**
(21) Application number: 06753161.6
(22) Date of filing: 01.06.2006
(51) Int. Cl.: A62D 3/02, A62D 101/02, C02F 3/34, C02F 11/00, C02F 11/02, C02F 101/36

(54) **METHOD OF DETOXICATION OF YPERITE BY USING HALOALKANE DEHALOGENASES**
..
PROCEDE DE DETOXICATION DE L'YPERITE DANS LEQUEL SONT UTILISEES DES HALOALKANES DESHALOGENASES

(30) Priority: 03.06.2005 CZ 20050352
(43) Date of publication of application: 19.03.2008
(73) Proprietor: Masarykova univerzita, 601 77 Brno (CZ)
(72) Inventor: PROKOP, Zbynek, 612 00 Brno (CZ); DAMBORSKY, Jiri, 628 00 Brno (CZ); OPLUSTIL, Frantisek, 628 00 Brno (CZ); JESENSKA, Andrea, 618 00 Brno (CZ); NAGATA, Yuji, Aoba-ku 980-0861Sendai (JP)
(74) Representative: Gabrielova, Marta
(86) International application number: PCT/CZ2006/000036
(87) International publication number: WO 2006/128390

(56) References cited:
- WO-A-01/56380
- WO-A-2004/066726

## Description

### Field of the Invention

This invention relates to method for detoxification of yperite 2,2'-dichlorodiethylsulfide - by using haloalkane dehalogenases (Enzyme Commission number EC 3.8.1.5) as a primary, chemically active component of decontamination compositions. Decontamination compositions are designated for detoxification of yperite (2,2-dichlorodiethylsulfide) on the surface of military hardware, transportation, industrial and agricultural hardware, technical devices and constructional objects (hereafter instrumentation), human or animal skin and elements of environment (water, soil, sediments and air), that are contaminated by this highly toxic blistering substance.

### State of the Art

At the present time, decontamination compositions are being used in the armed forces, civil defense troops, fire services and rescue forces, that exhibit high unit consumption and undesirable aggressiveness on material, because their chemically active components are stechiometric agents, that are gradually consumed during their reaction with yperite. Their application on instrumentation leads to depreciation of decontaminated material or surfaces by corrosion and if these compositions get into soil or water, it endangers environment.

There have been described enzymes in the literature that exhibit activity against highly toxic organophosphorous (neural) substances, called organophosphate hydrolases, OPA anhydrases or DFPases. As the only example of biological detoxification of blistering yperite (2,2'-dichlorodiethylsulfide), the use of bacteria species *Rhodococcus rhodochrous* IGTS8 (ATCC 53968) was mentioned in the art so far, which has the ability to utilize a chemical analog of yperite 2-chloroethyl-ethylsulfide as the only source of carbon for its growth [Kilbane, J. J., and Jackowski, K. (1996) J. Chem. Tech. Biotechnol. 65, 370-374]. Detoxification activity of bacteria species *Rhodococcus rhodochrous* IGTS8 (ATCC 53968) is based on splitting the S-C bond in the molecule. The application of the enzyme splitting C-S bond in a non-toxic product of hydrolysis, thiodiglycol, has been published [Harvey, S., DeFrank, J. J., Valdes, J. J., Kamely, D, and Chakrabarty, A. M., (1990) Proceedings: Biotechnology-Biodegradation Workshop Symposium by US Army Research Office, 47-58; Kilbane, J. J., (1990) Resources Conserv. and Recycl. 3, 69-79].

Haloalkane dehalogenases are enzymes that are able to remove halogen from halogenated aliphatic compound by a hydrolytic replacement, forming the corresponding alcohols [Janssen, D. B. Pries, F., and Van der Ploeg, J. R. (1994) Annual Review of Microbiology 48, 163-191]. Hydrolytic dehalogenation proceeds by formal nucleophilic substitution of the halogen atom by hydroxyl ion. Structurally, haloalkane dehalogenases belong to the α/β-hydrolase fold superfamily [Ollis, D. L., Cheah, E., Cygler, M., Dijkstra, B., Frolow, F., Franken, S. M., Harel, M., Remington, S. J., Silman, I., Schrag, J., Sussman, J. L., Verschueren, K. H. G., and Goldman, A. (1992) Protein Engineering 5, 197-211]. Haloalkane dehalogenases contain a nucleophile elbow [Damborsky, J. (1998) Pure and Applied Chemistry 70, 1375-1383] that belongs to the most conserved structural features of the α/β-hydrolase fold. Another highly conserved region in haloalkane dehalogenases is the central β-sheet that is flanked on both sides by α-helixes that form hydrophobic core of the main domain. The main domain carries the catalytic triad: aspartic acid - histidine - aspartic acid/glutamic acid (Asp-His-Asp/Glu). The second domain is solely consisting of α-helixes and is placed like a cap on top of the main domain. The interface between the main and the cap domain forms the active site of the enzyme. Whereas there is significant similarity between main domains, the sequence and structure of the cap domain varies significantly in different haloalkane dehalogenases. It is supposed, that character and structure of the cap domain essentially determine the substrate specificity [Pries, F., Van den Wijngaard, A. J., Bos, R., Pentenga, M., and Janssen, D. B. (1994) Journal of Biological Chemistry 269, 17490-17494; Kmunicek, J., Luengo, S., Gago, F., Ortiz, A. R., Wade, R. C., and Damborsky, J. (2001) Biochemistry 40, 8905-8917].

### Description of the invention

The disadvantages of the above mentioned existing decontamination compositions containing stechiometric agents, are to the great extent overcome by preparations in accordance with the present invention comprising a catalyst of hydrolytic detoxification of yperite (2,2'-dichlorodiethylsulfide), which is enzyme or mixture of enzymes haloalkane dehalogenases. The basic and active component of the compositions is at least one wild type or modified enzyme of the haloalkane dehalogenase family (EC 3.8.1.5). The present invention provides a method of detoxication of yperite by the use of haloalkane dehalogenases which comprises hydrolytic dehalogenation of yperite, wherein yperite is treated with the detoxification composition containing one or more wild type or modified haloalkane dehalogenases and is thereby converted to the non-toxic product thiodiglycol.

Haloalkane dehalogenase is expressed in the natural producer or in a heterologous host organism, e.g. in bacteria *Escherichia coli,* or in yeast *Pichia pastoris,* The enzyme used can be comprised in non-living or living cells, in the form of crude extract or purified protein. As an enzyme for the dehalogenase composition at least one haloalkane dehalogenase selected from the family of enzymes EC 3.8.1.5 is used, e.g. haloalkane dehalogenase DhaA from *Rhodococcus rhodochrous* NCIMB 13064, DmbA from *Mycobacterium bovis* 5033/66 or LinB from *Sphingomonas paucimobilis* UT26. Preferably, a wild type haloalkane dehalogenase or modified polypeptide with haloalkane dehalogenase activity having an amino acid sequence that corresponds at least in 80 %, more preferably at least in 90 %, to the sequence No. 2, 4 or 6 (amino acid sequences of LinB, DhaA and DmbA, respectively) or the mixture of the wild type haloalkane dehalogenase and the modified polypeptide is used.

Haloalkane dehalogenases constitute an important group of enzymes that are able to cleave the halogen-carbon bond in halogenated aliphatic compounds. They exhibit a broad substrate specificity including haloalkanes, halocycloalkanes, haloalkenes, haloethers and haloalcohols. The mechanism of dehalogenation is based on the nucleophilic attack to the carbon atom to which the halogen is bound and proceeds to cleavage of halogen ion and formation of alkyl-enzyme intermediate. The intermediate is subsequently hydrolyzed, yielding the corresponding alcohol, halogen ion and proton. The enzyme haloalkane dehalogenase transforms yperite into non-toxic bis(2-hydroxyethyl)sulfide by hydrolytic dehalogenation.

In the decontamination compositions haloalkane dehalogenase can be in crude extract or purified, immobilized on a carrier material, free in aqueous solution, in a monophasic organic or aqueous solution or in organic/aqueous biphasic systems. Enzymes can be immobilized by absorption on the inorganic or organic carrier material (such as: Celite, activated charcoal, aluminium oxide, cellulose, synthetic resins or Sephadex which is based on synthetically derived polysaccharide (dextran) or covalent attachment onto the surface of organic material (such as: cellulose, dextran, starch, chitin, agarose) or inorganic material (such as: porous glass), or synthetic polymeric carrier material (such as: VA-Epoxy Biosynt, Eupergit): The enzyme may be immobilized also by cross-linking (linkage to each other) or entrapping enzyme into a solid matrix or a compartment confined by a membrane.

The enzyme haloalkane dehalogenase may be dissolved, crystalline, lyophilized or precipitated. The liquid medium is an organic solvent, a mono-phasic aqueous solution of organic solvent or bi-phasic system consisting of organic solvent and water. The enzyme can be confined to a restricted area, where it remains catalytically active - entrapped into a solid matrix or into compartments restricted by a membrane. Enzymes may be entrapped into a biological matrix, e.g., agar gel, alginate gel, κ-carragenan. The enzyme can be entrapped also to inorganic stable matrices, e.g., silica gel. A tight network that is able to carry isolated enzyme can be obtained by polymerization of synthetic monomers, e.g., polyacrylamide, in the presence of the enzyme. Depending on the immobilization technique, the properties of the enzyme such as catalytic rate, stability and binding affinity may be significantly altered. The hydrolytic detoxification of yperite catalysed by the enzyme can be performed in the temperature range of 10 - 70 °C with reaction optimum of about 40 °C.

Additional components are aqueous buffer systems (e.g., phosphate buffer, tris-sulfate buffer, glycine buffer, acetate buffer or citrate buffer) which stabilize the neutral pH being close to optimum interval of 7.0 - 8.5. The pH activity profile is broader and allows pH interval from 4 to 12 while maintaining a reasonable activity. Other additional components are surfactants or organic solvents that facilitate dissolving of yperite in aqueous solvents. Addition of water-miscible organic solvents, e.g., methanol, tert-butanol, acetone, dioxane, acetonitrile, dimethyl formamide, dimethyl sulfoxide, tetrahydrofuran, 3-methyl-3-pentanol and pyridine, can be used at concentration up to 70 % of the total volume depending on the enzyme stability.

Decontamination compositions based on haloalkane dehalogenases can consist of two macroscopic phases, namely the aqueous phase containing the dissolved enzyme and a second phase consisting of organic solvents, partially water soluble or insoluble in water, e.g., ethyl acetate, diethyl ether, methyl tert-butyl ether, cyclohexanol, n-propylacetate, ethyl chloroacetate, bis(2-chloroethyl)ether, isopropyl acetate, butyl acetate, isobutyl acetate, hexanol, isoamyl acetate, n-amyl acetate, toluene, octanol, isoheptane, n-butyl ether, cyclohexane, 2-methylpentane, n-hexane, methylcyclohexane and n-octane. Organic phase enhances solubility of yperite in the decontamination composition which penetrates into water phase. The reaction takes place in aqueous phase, where the enzyme is in natural environment and is not in direct contact with organic solvent, where the most of dissolved yperite is located. The transfer of reactant and product between the two phases, reactant to the enzyme, product from the enzyme, can be increased by enlarging the surface between the two phases (producing a fine dispersion) or by stirring. The bulk water can be replaced by addition of water immiscible organic solvent. The enzyme is then suspended in a monophasic organic solvent. The optimum catalytic activity of the enzyme in organic solvent can be obtained by adjustment and maintenance of the water content. This can be conventionally obtained by a pair of salt/hydrate, e.g., CaCl₂ • H₂O/2 H₂O, Nal anh./2 H₂O, Na₂HPO₄ anh./2 H₂O, NaOAc anh./3 H₂O, NaBr anh./2 H₂O, Na₄P₂O₇ anh./7 H₂O, Na₂HPO₄ • 2 H₂O/7 H₂O, Na₂SO₄ anh./10 H₂O, Na₂HPO₄ • 7 H₂O/12 H₂O, that are added to the solvent and function as a water content buffer. The enzyme solubility in lipophilic organic solvents can be modified by covalent attachment of the amphiphatic polymer polyethylene glycol to the surface of enzyme. Linkage of the polymer chain onto the enzyme surface is achieved by reaction of ε-amino groups of lysine residues with "linker", e.g., cyanuric chloride. Protein stabilizers such as polyalcohols, e.g., sugar alcohols or glycerol, inactivated proteins, e.g., bovine serum albumin, or polymers, which show a certain structural resemblance with water, e.g., polyethylene glycol, polyvinyl alcohol, can be added to the reaction medium to enhance the enzyme stability.

Haloalkane dehalogenases used according to this invention can be further produced by means of rational design based on structural analysis, e.g., protein crystallography, nuclear magnetic resonance and circular dichroism spectroscopy, and biochemical characterization, e.g., steady-state kinetics, transient-state kinetics, stability and thermo stability assays, spectroscopic analyses and the like, followed by computer modelling, e.g., sequence comparisons, phylogenetic analysis, homology modelling, molecular docking, molecular mechanics, molecular dynamics, quantum mechanics and multivariate statistics, and DNA mutagenesis, e.g., cassette mutagenesis, site-directed mutagenesis, chemical mutagenesis, error-prone PCR, site saturation mutagenesis, ensemble mutagenesis, recursive ensemble mutagenesis, scanning saturation mutagenesis, mutator strains, etc. The procedure includes altering at least one amino acid residue of the haloalkane dehalogenase with another amino acid residue or recombining two or more members of the haloalkane dehalogenases to obtain a modified enzyme with improved efficacy. Modified nucleic acids can be introduced into a cell, in which they can be expressed to provide an altered haloalkane dehalogenase.

Advantages of decontamination compositions with haloalkane dehalogenases are that
a) It possesses desired detoxification activity towards the yperite, which is homogeneously dissolved in aqueous solutions with pH in the range from 4 to 12 (with the neutral optimum of pH 7.0 to 8.5) at the temperature range +10 to +70°C with reaction optimum at about +40 °C.
b) A low initial concentration (1×10⁻⁶ mol.l⁻¹) is sufficient to attain desired reaction rate that means a satisfactory level of detoxification in about 15 to 30 minutes, whereas the upper limit of the enzyme concentration is 1×10⁰ mol.l⁻¹.
c) It exhibits catalytic activity, i.e. it remains unexhausted during reaction, which brings cost reduction in logistic area.
d) It doesn't exhibit chemical aggressiveness against common construction materials and components of technique that resist the affect of neutral aqueous, corrosively non-aggressive decontamination compositions (aqueous suspensions, foams, emulsions or micro-emulsions).
e) It doesn't exhibit any toxicity and is degradable in environment

### Description of the Exemplary Embodiment

The enzyme is prepared by homologous expression in native producer or by heterologous expression in a host organism, e.g., bacterium *Escherichia coli* or yeast *Pichia pastoris.* According to the invention, the enzyme present in living or non-living cells is used in the form of crude extract or purified protein.

### Example 1

To overproduce wild type of enzyme haloalkane dehalogenase LinB from *Sphingomonas paucimobilis* UT26 (Sequence 1 and 2) the corresponding gene was cloned in the pPICZαA expression vector. Cloned plasmids were then transferred into *Pichia pastoris* GS115. *Pichia pastoris* GS115 was then cultured at 28 °C in growth medium (1 weight % of yeast extract, 2 weight % of peptone, 4×10⁻⁵ weight % of Biotine and 1 weight % of casamino acid in 100 mM potassium phosphate buffer, pH 6.5). The induction of the enzyme synthesis was initiated by addition of 0.7 volume % of methanol when the culture reached an optical density of 2 at 600 nm. After induction the culture was incubated at 28 °C for 10 h and then harvested. Ammonium sulfate was added to supernatant to a final concentration of 75 % of saturation. Solution was stirred 30 min until the added ammonium sulfate was dissolved. The supernatant was centrifugated 15 min at 11 000 g. Pellet was than re-suspended in 20 mM potassium phosphate buffer, pH 7.5 with content of 0.5 M sodium chloride and 10 mM imidazole. The haloalkane dehalogenase was then purified on a Ni-NTA Sepharose column HR 16/10 (Qiagen, Germany). The His-tagged haloalkane dehalogenase was bound to the resin placed in the equilibrating buffer, which contained 20 mM potassium phosphate buffer pH 7.5; 0.5 M sodium chloride and 10 mM imidazole. Unbound and weakly bound proteins were washed off by buffer containing 60 mM imidazole. Then the His-tagged enzyme LinB was eluted by buffer containing 160 mM imidazole. The active fractions were dialyzed overnight against 50 mM potassium phosphate buffer, pH = 7.5. The enzyme was stored at 4 °C in 50 mM potassium phosphate buffer, pH 7.5, containing 10 % glycerol and 1 mM 2-mercaptoethanol to enhance long-lasting enzyme stability.

Hydrolytic dehalogenation catalyzed by haloalkane dehalogenase (wild type or modified) converts the toxic yperite into non-toxic bis(2-hydroxyethyl)sulfide. The hydrolytic dehalogenation of yperite was catalyzed by haloalkane dehalogenase at 37 °C in 50 mM phosphate buffer (pH 7.5; adjusted by addition of 1M NaOH solution). The yperite was added into reaction buffer to its final concentration of 94.3 mg.l⁻¹ of yperite in buffer. Reaction was initiated by addition of solution of enzyme LinB (50 mM potassium phosphate buffer pH 7.5; 1×10⁻⁵ to 1×10⁻⁴ mol.l⁻¹ of haloalkane dehalogenase LinB, 10 vol % of glycerol and 1 mmol.l⁻¹ 2-mercaptoethanol). Operation of haloalkane dehalogenase leads to rapid and complete decontamination of the yperite. The kinetics of reaction is shown in Figure 1. Catalytic activity/power of haloalkane dehalogenase LinB at decontamination of yperite is *k*_{cat}*lK*ₘ *=* 6.9 s⁻¹.mM⁻¹.

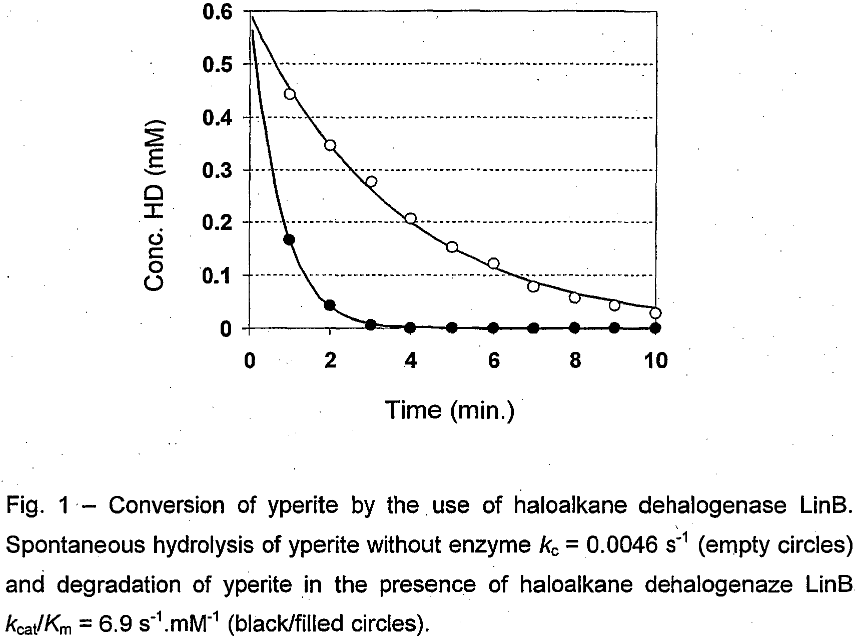

Sequence 1 and 2. Sequence of the gene *linB* and haloalkane dehalogenase LinB isolated from bacterium *Sphingomonas paucimobilis* UT26.

### Example 2

To overproduce enzyme haloalkane dehalogenase DhaA from *Rhodococcus rhodochrous* NCIMB 13064 (Sequence 3 and 4), the corresponding gene was cloned in the pAQN vector containing *tac* promotor (P_{tac}) under the control of *lacl*^{q}*. Escherichia coli* BL21 containing pAQN plasmid was cultured in 250 ml Luria broth at 37 °C. The induction of enzyme synthesis was initiated by addition of isopropyl-β-D-thiogalactopyranoside to a final concentration of 0.5 mM when the culture reached an optical density of 0.6 at 600 nm. After induction, the culture was incubated at 30°C for 4 h and then harvested. The cells were disrupted by sonication using a Soniprep 150 (Sanyo, UK). The supernatant was used after centrifugation at 100 000 *g* for 1 h. The haloalkane dehalogenase was purified on a Ni-NTA Sepharose column HR 16/10 (Qiagen, Germany). His-tagged haloalkane dehalogenase was bound onto the resin placed in the equilibrating buffer, which contained 20 mM potassium phosphate buffer pH 7.5, 0.5 M sodium chloride and 10 mM imidazole. Unbound and weakly bound proteins were washed off by buffer containing 60 mM imidazole. The His-tagged haloalkane dehalogenase was then eluted by buffer containing 160 mM imidazole. The active fractions were dialysed overnight against 50 mM potassium phosphate buffer, pH 7.5. The enzyme was stored at 4 °C in 50 mM potassium phosphate buffer, pH 7.5, containing 10 % glycerol and 1mM 2-mercaptoethanol enhancing long-term enzyme stability.

Enzyme haloalkane dehalogenase DhaA or its variant is the part of decontamination composition that contains aforementioned enzyme at concentration 1×10⁻⁶ to 1×10⁻⁴ mol.l⁻¹, further 1 to 5 vol % of aliphatic hydrocarbon of general formula CₙH₂ₙ₊₂ or cyclic aliphatic hydrocarbon of general formula CₙH₂ₙ, where n is 6 to 12, further 5 to 20 vol % of aliphatic alcohol of general formula CₙH₂ₙ₊₁OH, where n is 2 to 4, further 3 to 15 weight % of anion active tenside of general formula CₙH₂ₙ₊₁OSO₃Me, where n is 10 to 16 and Me stands for counter ion (Na⁺, K⁺ or monoethanol amonium), 1 to 10 weight % alkylbenzenesulfonate of general formula R⁽³⁾-(Ar)SO₃⁻.Me⁺, where R⁽³⁾ stands for alkyl with 11 to 13 atoms of carbon, and Me⁺ indicates sodium ion, further components of glycine buffer to adjust pH of aqueous solution in the range from 7 to 9, or else glycine of total concentration 0.1 mol.l⁻¹, etc. Required pH 8.2 is reached by addition of 1 M NaOH. The rest to make 100% is water. The catalytic power of haloalkane dehalogenase DhaA at decontamination of yperite is *k*_{cat}/*K*ₘ= 5.7 s⁻¹.mM⁻¹.

Sequence 3 and 4. Sequence of the gene *dhaA* and haloalkane dehalogenase DhaA isolated from bacterium *Rhodococcus rhodochrous* NCIMB 13064.

### Example 3

To overproduce enzyme haloalkane dehalogenase DmbA from *Mycobacterium bovis* 5033/66 (Sequence 5 and 6), the same procedure as in example 1 is followed. Enzyme haloalkane dehalogenase DmbA or its variant is the part of decontamination composition that contains aforementioned enzyme at concentrations 1×10⁻⁶ to 1×10⁻⁴ mol.l⁻¹, further 1 to 20 vol % of aliphatic alcohol of general formula CₙH₂ₙ₊₁OH, where n is 2 to 4, further 3 to 15 weight % of anion active surfactant of general formula CₙH₂ₙ₊₁OSO₃Me, where n is 10 to 16 and Me stands for counter ion (Na⁺, K⁺ or monoethanol amonium), 1 to 10 weight % of ethoxy nonylphenol of general formula C₉H₁₉-Ar-O-(C₂H₄O)ₙH, where n is 9 to 10, further components of glycine buffer to adjust pH of aqueous solution in the range from 7 to 9, or else glycine of total concentration 0.1 mol.l⁻¹, etc. Required pH is reached by addition of 1M NaOH. The rest to make 100% is water. The catalytic power of haloalkane dehalogenase DmbA at decontamination of yperite is *k*_{cat}/*K*m= 6.0 s⁻¹.mM⁻¹.

Sequence 5 and 6. Sequence of the gene *dmbA* and haloalkane dehalogenase DmbA isolated from bacterium *Mycobacterium bovis* 5033/66.

### Example 4

Enzyme haloalkane dehalogenase LinB or its variant is the part of decontamination composition that contains the enzyme at concentrations 1×10⁻⁶ to 1×10⁻⁴ mol.l⁻¹, further 1 to 15 weight % of anion active surfactant of general formula CₙH₂ₙ₊₁OSO₃Me, where n is 10 to 16 and Me stands for counter ion (Na⁺, K⁺ or monoethanol amonium), 1 to 10 weight % of ethoxylated nonylphenol of general formula C₉H₁₉-Ar-O-(C₂H₄O)ₙH, where n is 9 to 10, further components of phosphate buffer to adjust pH of aqueous solution in the range from 7 to 8.5; that is KH₂PO₄ a K₂HPO₄ in the required ratio and in a total concentration 50 mmol.l⁻¹, etc. The rest to make 100% is water.

### Industrial Applicability

This invention is utilizable in industry to eliminate yperite from the surfaces of military hardware, transportation, industrial and agricultural hardware, technical devices and constructional objects, of the human or animal skin and elements of environment, that are contaminated by this highly toxic blistering substance. This technology is utilizable in armed forces and also in civil services, generally there, where the use of detoxication compositions to decontaminate blistering substances is necessary.

## Claims

1. Method of detoxication of yperite by the use of haloalkane dehalogenases **characterized in that** said yperite is treated with at least one haloalkane dehalogenase selected from the group of enzymes EC 3.8.1.5, at the concentration of enzyme 1×10⁻⁶ to 1×10⁰ mol.l⁻¹, at temperature from +10 °C to +70 °C and pH from 4 to 12 in liquid medium.

2. Method according to claim 1, **characterized in that** the haloalkane dehalogenase is at least one enzyme selected from the group comprising dehalogenases LinB from *Sphingomonas paucimobilis* UT26 (Sequence 1 and 2), DhaA from *Rhodococcus rodochrous* NCIMB 13064 (Sequence 3 and 4), DmbA from *Mycobacterium bovis* 5033/66 (Sequence 5 and 6).

3. Method according to claim 1 or 2, **characterized in that** the haloalkane dehalogenase is wild type haloalkane dehalogenase or modified polypeptide with haloalkane dehalogenase activity having an amino acid sequence that corresponds at least in 80% to the sequence No. 2, 4 or 6 or the mixture of the wild type haloalkane dehalogenase and the modified polypeptide.

4. Method according to claim 1 or 2, **characterized in that** the haloalkane dehalogenase is wild type haloalkane dehalogenase or modified polypeptide with haloalkane dehalogenase activity having an amino acid sequence that corresponds at least in 90% to the sequence No. 2, 4 or 6 or the mixture of the wild type haloalkane dehalogenase and the modified polypeptide.

5. Method according to claims 1 to 4, **characterized in that** the liquid medium is an organic solvent, a mono-phasic aqueous solution of organic solvent or biphasic system consisting of organic solvent and water.

6. Method according to claim 5, **characterized in that** the haloalkane dehalogenase solution includes a buffer system.

7. Method according to claims 1 to 6, **characterized in that** the preparation of haloalkane dehalogenase is carried out in the presence of at least one protein stabilizer, selected from the group consisting of polyalcohols, inactive proteins, polymers.

8. Method according to claims 1 to 7, **characterized in that** the enzyme haloalkane dehalogenase is used as soluble form or as crystalline form or as lyophilized form or as precipitated form.

9. Method according to claims 1 to 8, **characterized in that** the enzyme haloalkane dehalogenase is immobilized by adsorption, by ionic binding or by covalent attachment onto the surface of organic or inorganic carrier.

10. Method according to claims 1 to 8, **characterized in that** the enzyme haloalkane dehalogenase is immobilized by cross-linking or entrapping enzyme into a solid matrix or a compartment confined by a membrane.

11. Method according to claim 1 to 10, **characterized in that** the detoxication is carried out in the presence of surfactants.

## Patentansprüche

1. Verfahren zur Yperit-Detoxikation unter Einsatz von Haloalkan-Dehalogenase **dadurch gekennzeichnet, dass** auf das Yperit mindestens eine Haloalkan-Dehalogenase, ausgewählt aus der Enzym-Gruppe EC 3.8.1.5, unter einer Enzym-Konzentration im Bereich von 1×10⁻⁶ bis 1×10⁰ mol.l⁻¹, bei einer Temperatur von +10°C bis +70°C und beim pH-Wert von 4 bis 12 im flüssigen Medium einwirkt.

2. Verfahren nach dem Anspruch 1 **dadurch gekennzeichnet, dass** die Haloalkan-Dehalogenase mindestens ein Enzym ist, ausgewählt aus der Gruppe umfassend Dehalogenase LinB aus *Sphingomonas paucimobilis* UT26 (Sequenz 1 und 2), DhaA aus *Rhodococcus rodochrous* NCIMB 13064 (Sequenz 3 und 4), DmbA aus *Mycobacterium bovis* 5033/66 (Sequenz 5 und 6).

3. Verfahren nach dem Anspruch 1 oder 2 **dadurch gekennzeichnet, dass** die Haloalkan-Dehalogenase eine Haloalkan-Dehalogenase wilden Typs oder ein modifiziertes Polypeptid mit einer Haloalkan-Dehalogenase-Aktivität mit Aminosäure-Sequenz ist, entsprechend mindestens von 80% der Sequenz Nr. 2, 4 oder 6, oder ein Gemisch von Haloalkan-Dehalogenase wilden Typs und des modifizierten Polypeptids.

4. Verfahren nach dem Anspruch 1 oder 2 **dadurch gekennzeichnet, dass** die Haloalkan-Dehalogenase eine Haloalkan-Dehalogenase wilden Typs oder ein modifiziertes Polypeptid mit einer Haloalkan-Dehalogenase-Aktivität mit Aminosäure-Sequenz ist, entsprechend mindestens von 90% der Sequenz Nr. 2, 4 oder 6, oder ein Gemisch von Haloalkan-Dehalogenase wilden Typs und des modifizierten Polypeptids.

5. Verfahren nach den Ansprüchen 1 bis 4 **dadurch gekennzeichnet, dass** das flüssige Medium ein organisch Lösungsmittel, eine wässrige Einphasenlösung des organischen Lösungsmittels oder ein Zweiphasensystem bestehend aus organischen Lösungsmittel und Wasser ist.

6. Verfahren nach dem Anspruch 5 **dadurch gekennzeichnet, dass** die Lösung von Haloalkan-Dehalogenase ein Puffersystem umfasst.

7. Verfahren nach den Ansprüchen 1 bis 6 **dadurch gekennzeichnet, dass** die Zubereitung der Haloalkan-Dehalogenase in Anwesenheit mindestens eines Proteinstabilisators durchgeführt wird, ausgewählt aus der Gruppe umfassend Polyalkohole, inaktive Proteine, Polymere.

8. Verfahren nach den Ansprüchen 1 bis 7 **dadurch gekennzeichnet, dass** das Enzym Haloalkan-Dehalogenase in löslicher Form oder in kristalliner Form oder in lyophilisierter Form oder in koagulierter Form zum Einsatz kommt.

9. Verfahren nach den Ansprüchen 1 bis 8 **dadurch gekennzeichnet, dass** das Enzym Haloalkan-Dehalogenase durch die Adsorption, die Ionenbindung oder kovalente Bindung auf der Oberfläche des organischen oder anorganischen Trägers immobilisiert ist.

10. Verfahren nach den Ansprüchen 1 bis 8 **dadurch gekennzeichnet, dass** das Enzym Haloalkan-Dehalogenase durch Cross-Linking oder das Einschliessen des Enzyms in einer Festmatrize oder in einem durch eine Membrane abgegrenzten Raum immobilisiert ist.

11. Verfahren nach den Ansprüchen 1 bis 10 **dadurch gekennzeichnet, dass** die Detoxikation in Anwesenheit von Surfaktanten erfolgt.

## Revendications

1. Procédé de détoxication de l'ypérite par l'utilisation des halogénoalcanes déshalogénases, **caractérisé en ce que** l'ypérite est traité par au moins une halogénoalcane déshalogénase sélectionnée parmi un groupe d'enzymes EC 3.8.1.5 à la concentration de l'enzyme de 1×10⁻⁶ à 1×10⁰ mol.l⁻¹, à la température de +10 °C jusqu'à +70 °C et au pH de 4 à 12 en milieu liquide.

2. Procédé selon la revendication 1, **caractérisé en ce que** la halogénoalcane déshalogénase est au moins une enzyme sélectionnée parmi un groupe comprenant la déshalogénase LinB de *Sphingomonas paucimobilis* UT26 (Séquence 1 et 2), DhaA de *Rhodococcus rodochrous* NCIMB 13064 (Séquence 3 et 4), DmbA de *Mycobacterium bovis* 5033/66 (Séquence 5 et 6).

3. Procédé selon la revendication 1 ou 2, **caractérisé en ce que** la halogénoalcane déshalogénase est une halogénoalcane déshalogénase de type sauvage ou un polypeptide modifié avec activité d'halogénoalcane déshalogénase ayant une séquence d'acides aminées, laquelle correspond au moins à 80% à la séquence n° 2, 4 ou 6, ou un mélange de la halogénoalcane déshalogénase de type sauvage et du polypeptide modifié.

4. Procédé selon la revendication 1 ou 2, **caractérisé en ce que** la halogénoalcane déshalogénase est une halogénoalcane déshalogénase de type sauvage ou un polypeptide modifié avec activité d'halogénoalcane déshalogénase ayant une séquence d'acides aminées, laquelle correspond au moins à 90% à la séquence n° 2, 4 ou 6, ou un mélange de la halogénoalcane déshalogénase de type sauvage et du polypeptide modifié.

5. Procédé selon les revendications 1 à 4, **caractérisé en ce que** le milieu liquide est un solvant organique, une solution aqueuse monophasée du solvant organique ou un système biphasé consistant du solvant organique et de l'eau.

6. Procédé selon la revendication 5, **caractérisé en ce que** la solution de l'halogénoalcane déshalogénase contient un système tampon.

7. Procédé selon les revendications 1 à 6, **caractérisé en ce que** la préparation de l'halogénoalcane déshalogénase s'effectue en présence d'au moins un stabilisateur des protéines sélectionné parmi un groupe consistant de polyalcools, de protéines inactifs, de polymères.

8. Procédé selon les revendications 1 à 7, **caractérisé en ce que** l'enzyme halogénoalcane déshalogénase est utilisée sous forme soluble ou sous forme cristalline ou sous forme lyophilisée ou sous forme précipitée.

9. Procédé selon les revendications 1 à 8, **caractérisé en ce que** l'enzyme halogénoalcane déshalogénase est immobilisée par adsorption, par liaison ionique ou par liaison covalente sur la surface du support organique ou inorganique.

10. Procédé selon les revendications 1 à 8, **caractérisé en ce que** l'enzyme halogénoalcane déshalogénase est immobilisée par un cross-linking ou par la fermeture de l'enzyme dans une matrice solide ou dans un compartiment confiné par une membrane.

11. Procédé selon les revendications 1 à 10, **caractérisé en ce que** la détoxication s'effectue en présence des surfactants.


### SEQUENCE LISTING

<110> Masarykova univerzita
<120> Method of detoxication of yperite by using haloalkane dehalogenases
<130> D0096N/PCT
<150> CZ 2005-352
   <151> 2005-06-03
<160> 6
<170> PatentIn version 3.3
<210> 1
   <211> 891
   <212> DNA
   <213> Sphingomonas paucimobilis
<400> 1
   atgagcctcg gcgcaaagcc atttggcgag aagaaattca ttgagatcaa gggccggcgc      60
   atggcctata tcgatgaagg gaccggcgat ccgatcctct tccagcacgg caatccgacg     120
   tcgtcctatc tgtggcgcaa tatcatgccg cattgcgccg ggctgggacg gctgatcgcc     180
   tgtgacctga tcggcatggg cgattcggac aagctcgatc cgtcggggcc cgagcgttat     240
   gcctatgccg agcatcgtga ctatctcgac gcgctgtggg aggcgctcga tctcggggac     300
   agggttgttc tggtcgtgca tgactggggg tccgccctcg gcttcgactg ggcccgccgc     360
   caccgcgagc gtgtacaggg gattgcctat atggaagcga tcgccatgcc gatcgaatgg     420
   gcggattttc ccgaacagga tcgcgatctg tttcaggcct ttcgctcgca ggcgggcgaa     480
   gaattggtgt tgcaggacaa tgtttttgtc gaacaagttc tccccggatt gatcctgcgc     540
   cccttaagcg aagcggagat ggccgcctat cgcgagccct tcctcgccgc cggcgaagcc     600
   cgtcgaccga ccctgtcttg gcctcgccaa atcccgatcg caggcacccc ggccgacgtg     660
   gtcgcgatcg cccgggacta tgccggctgg ctcagcgaaa gcccgattcc gaaactcttc     720
   atcaacgccg agccgggagc cctgaccacg ggccgaatgc gcgacttctg ccgcacatgg     780
   ccaaaccaga ccgaaatcac ggtcgcaggc gcccatttca tccaggagga cagtccggac     840
   gagattggcg cggcgattgc ggcgtttgtc cggcgattgc gcccagcata a              891
<210> 2
   <211> 296
   <212> PRT
   <213> Sphingomonas paucimobilis
<400> 2
   Met Ser Leu Gly Ala Lys Pro Phe Gly Glu Lys Lys Phe Ile Glu Ile
   1 5 10 15
   Lys Gly Arg Arg Met Ala Tyr Ile Asp Glu Gly Thr Gly Asp Pro Ile
   20 25 30
   Leu Phe Gln His Gly Asn Pro Thr Ser Ser Tyr Leu Trp Arg Asn Ile
   35 40 45
   Met Pro His Cys Ala Gly Leu Gly Arg Leu Ile Ala Cys Asp Leu Ile
   50 55 60
   Gly Met Gly Asp Ser Asp Lys Leu Asp Pro Ser Gly Pro Glu Arg Tyr
   65 70 75 80
   Ala Tyr Ala Glu His Arg Asp Tyr Leu Asp Ala Leu Trp Glu Ala Leu
   85 90 95
   Asp Leu Gly Asp Arg Val Val Leu Val Val His Asp Trp Gly Ser Ala
   100 105 110
   Leu Gly Phe Asp Trp Ala Arg Arg His Arg Glu Arg Val Gln Gly Ile
   115 120 125
   Ala Tyr Met Glu Ala Ile Ala Met Pro Ile Glu Trp Ala Asp Phe Pro
   130 135 140
   Glu Gln Asp Arg Asp Leu Phe Gln Ala Phe Arg Ser Gln Ala Gly Glu
   145 150 155 160
   Glu Leu Val Leu Gln Asp Asn Val Phe Val Glu Gln Val Leu Pro Gly
   165 170 175
   Leu Ile Leu Arg Pro Leu Ser Glu Ala Glu Met Ala Ala Tyr Arg Glu
   180 185 190
   Pro Phe Leu Ala Ala Gly Glu Ala Arg Arg Pro Thr Leu Ser Trp Pro
   195 200 205
   Arg Gln Ile Pro Ile Ala Gly Thr Pro Ala Asp Val Val Ala Ile Ala
   210 215 220
   Arg Asp Tyr Ala Gly Trp Leu Ser Glu Ser Pro Ile Pro Lys Leu Phe
   225 230 235 240
   Ile Asn Ala Glu Pro Gly Ala Leu Thr Thr Gly Arg Met Arg Asp Phe
   245 250 255
   Cys Arg Thr Trp Pro Asn Gln Thr Glu Ile Thr Val Ala Gly Ala His
   260 265 270
   Phe Ile Gln Glu Asp Ser Pro Asp Glu Ile Gly Ala Ala Ile Ala Ala
   275 280 285
   Phe Val Arg Arg Leu Arg Pro Ala
   290 295
<210> 3
   <211> 882
   <212> DNA
   <213> Rhodococcus rhodochrous
<400> 3
   atgtcagaaa tcggtacagg cttccccttc gacccccatt atgtggaagt cctgggcgag 60
   cgtatgcact acgtcgatgt tggaccgcgg gatggcacgc ctgtgctgtt cctgcacggt 120
   aacccgacct cgtcctacct gtggcgcaac atcatcccgc atgtagcacc gagtcatcgg 180
   tgcattgctc cagacctgat cgggatggga aaatcggaca aaccagacct cgattatttc 240
   ttcgacgacc acgtccgcta cctcgatgcc ttcatcgaag ccttgggttt ggaagaggtc 300
   gtcctggtca tccacgactg gggctcagct ctcggattcc actgggccaa gcgcaatccg 360
   gaacgggtca aaggtattgc atgtatggaa ttcatccggc ctatcccgac gtgggacgaa 420
   tggccggaat tcgcccgtga gaccttccag gccttccgga ccgccgacgt cggccgagag 480
   ttgatcatcg atcagaacgc tttcatcgag ggtgcgctcc cgaaatgcgt cgtccgtccg 540
   cttacggagg tcgagatgga ccactatcgc gagcccttcc tcaagcctgt tgaccgagag 600
   ccactgtggc gattccccaa cgagctgccc atcgccggtg agcccgcgaa catcgtcgcg 660
   ctcgtcgagg catacatgaa ctggctgcac cagtcacctg tcccgaagtt gttgttctgg 720
   ggcacacccg gcgtactgat ccccccggcc gaagccgcga gacttgccga aagcctcccc 780
   aactgcaaga cagtggacat cggcccggga ttgcactacc tccaggaaga caacccggac 840
   cttatcggca gtgagatcgc gcgctggctc cccgcactct ag 882
<210> 4
   <211> 293
   <212> PRT
   <213> Rhodococcus rhodochrous
<400> 4
   Met Ser Glu Ile Gly Thr Gly Phe Pro Phe Asp Pro His Tyr Val Glu
   1               5                   10                  15
   Val Leu Gly Glu Arg Met His Tyr Val Asp Val Gly Pro Arg Asp Gly
               20                  25                  30
   Thr Pro Val Leu Phe Leu His Gly Asn Pro Thr Ser Ser Tyr Leu Trp
           35                  40                  45
   Arg Asn Ile Ile Pro His Val Ala Pro Ser His Arg Cys Ile Ala Pro
       50                  55                  60
   Asp Leu Ile Gly Met Gly Lys Ser Asp Lys Pro Asp Leu Asp Tyr Phe
   65                  70                  75                  80
   Phe Asp Asp His Val Arg Tyr Leu Asp Ala Phe Ile Glu Ala Leu Gly
                   85                  90                  95
   Leu Glu Glu Val Val Leu Val Ile His Asp Trp Gly Ser Ala Leu Gly
               100                 105                 110
   Phe His Trp Ala Lys Arg Asn Pro Glu Arg Val Lys Gly Ile Ala Cys
           115                 120                 125
   Met Glu Phe Ile Arg Pro Ile Pro Thr Trp Asp Glu Trp Pro Glu Phe
       130                 135                 140
   Ala Arg Glu Thr Phe Gln Ala Phe Arg Thr Ala Asp Val Gly Arg Glu
   145                 150                 155                 160
   Leu Ile Ile Asp Gln Asn Ala Phe Ile Glu Gly Ala Leu Pro Lys Cys
                   165                 170                 175
   Val Val Arg Pro Leu Thr Glu Val Glu Met Asp His Tyr Arg Glu Pro
               180                 185                 190
   Phe Leu Lys Pro Val Asp Arg Glu Pro Leu Trp Arg Phe Pro Asn Glu
           195                 200                 205
   Leu Pro Ile Ala Gly Glu Pro Ala Asn Ile Val Ala Leu Val Glu Ala
       210                 215                 220
   Tyr Met Asn Trp Leu His Gln Ser Pro Val Pro Lys Leu Leu Phe Trp
   225                 230                 235                 240
   Gly Thr Pro Gly Val Leu Ile Pro Pro Ala Glu Ala Ala Arg Leu Ala
                   245                 250                 255
   Glu Ser Leu Pro Asn Cys Lys Thr Val Asp Ile Gly Pro Gly Leu His
               260                 265                 270
   Tyr Leu Gln Glu Asp Asn Pro Asp Leu Ile Gly Ser Glu Ile Ala Arg
   275 280 285
   Trp Leu Pro Ala Leu
   290
<210> 5
   <211> 903
   <212> DNA
   <213> Mycobacterium bovis
<400> 5
   atgacagcat tcggcgtcga gccctacggg cagccgaagt acctagaaat cgccgggaag 60
   cgcatggcgt atatcgacga aggcaagggt gacgccatcg tctttcagca cggcaacccc 120
   acgtcgtctt acttgtggcg caacatcatg ccgcacttgg aagggctggg ccggctggtg 180
   gcctgcgatc tgatcgggat gggcgcgtcg gacaagctca gcccatcggg acccgaccgc 240
   tatagctatg gcgagcaacg agactttttg ttcgcgctct gggatgcgct cgacctcggc 300
   gaccacgtgg tactggtgct gcacgactgg ggctcggcgc tcggcttcga ctgggctaac 360
   cagcatcgcg accgagtgca ggggatcgcg ttcatggaag cgatcgtcac cccgatgacg 420
   tgggcggact ggccgccggc cgtgcggggt gtgttccagg gtttccgatc gcctcaaggc 480
   gagccaatgg cgttggagca caacatcttt gtcgaacggg tgctgcccgg ggcgatcctg 540
   cgacagctca gcgacgagga aatgaaccac tatcggcggc cattcgtgaa cggcggcgag 600
   gaccgtcgcc ccacgttgtc gtggccacga aaccttccaa tcgacggtga gcccgccgag 660
   gtcgtcgcgt tggtcaacga gtaccggagc tggctcgagg aaaccgacat gccgaaactg 720
   ttcatcaacg ccgagcccgg cgcgatcatc accggccgca tccgtgacta tgtcaggagc 780
   tggcccaacc agaccgaaat cacagtgccc ggcgtgcatt tcgttcagga ggacagccca 840
   gaggaaatcg gtgcggccat agcacagttc gtccggcagc tccggtcggc ggccggcgtc 900
   tga 903
<210> 6
   <211> 300
   <212> PRT
   <213> Mycobacterium bovis
<400> 6
   Met Thr Ala Phe Gly Val Glu Pro Tyr Gly Gln Pro Lys Tyr Leu Glu
   1 5 10 15
   Ile Ala Gly Lys Arg Met Ala Tyr Ile Asp Glu Gly Lys Gly Asp Ala
   20 25 30
   Ile Val Phe Gln His Gly Asn Pro Thr Ser Ser Tyr Leu Trp Arg Asn
           35                  40                  45
   Ile Met Pro His Leu Glu Gly Leu Gly Arg Leu Val Ala Cys Asp Leu
       50                  55                  60
   Ile Gly Met Gly Ala Ser Asp Lys Leu Ser Pro Ser Gly Pro Asp Arg
   65                  70                  75                  80
   Tyr Ser Tyr Gly Glu Gln Arg Asp Phe Leu Phe Ala Leu Trp Asp Ala
                   85                  90                  95
   Leu Asp Leu Gly Asp His Val Val Leu Val Leu His Asp Trp Gly Ser
               100                 105                 110
   Ala Leu Gly Phe Asp Trp Ala Asn Gln His Arg Asp Arg Val Gln Gly
           115                 120                 125
   Ile Ala Phe Met Glu Ala Ile Val Thr Pro Met Thr Trp Ala Asp Trp
       130                 135                 140
   Pro Pro Ala Val Arg Gly Val Phe Gln Gly Phe Arg Ser Pro Gln Gly
   145                 150                 155                 160
   Glu Pro Met Ala Leu Glu His Asn Ile Phe Val Glu Arg Val Leu Pro
                   165                 170                 175
   Gly Ala Ile Leu Arg Gln Leu Ser Asp Glu Glu Met Asn His Tyr Arg
               180                 185                 190
   Arg Pro Phe Val Asn Gly Gly Glu Asp Arg Arg Pro Thr Leu Ser Trp
           195                 200                 205
   Pro Arg Asn Leu Pro Ile Asp Gly Glu Pro Ala Glu Val Val Ala Leu
       210                 215                 220
   Val Asn Glu Tyr Arg Ser Trp Leu Glu Glu Thr Asp Met Pro Lys Leu
   225                 230                 235                 240
   Phe Ile Asn Ala Glu Pro Gly Ala Ile Ile Thr Gly Arg Ile Arg Asp
                   245                 250                 255
   Tyr Val Arg Ser Trp Pro Asn Gln Thr Glu Ile Thr Val Pro Gly Val
               260                 265                 270
   His Phe Val Gln Glu Asp Ser Pro Glu Glu Ile Gly Ala Ala Ile Ala
           275                 280                 285
   Gln Phe Val Arg Gln Leu Arg Ser Ala Ala Gly Val
   290 295 300